# EUROPEAN PATENT SPECIFICATION

(11) **EP 0 821 925 B1**
(45) Date of publication and mention of the grant of the patent: **10.09.2003**
(21) Application number: 97112588.5
(22) Date of filing: 23.07.1997
(51) Int. Cl.: A61F 5/445

(54) **Ostomy bag**
Ostomiebeutel
Sac d'ostomie

(30) Priority: 01.08.1996 EP 96112399
(43) Date of publication of application: 04.02.1998
(73) Proprietor: Sealed Air Corporation (US), Saddle Brook, New Jersey 07663 (US)
(72) Inventor: Quacquarella, Cesare, 20146 Milano (IT); Buongiorno, Livio, 20090 Trezzano Sul Naviglio (Milan) (IT); Perego, Vittorio, 21052 Busto Arsizio (Varese) (IT)
(74) Representative: De Carli, Elda

(56) References cited:
- EP-A- 0 231 508
- EP-A- 0 433 060
- EP-A- 0 793 951
- GB-A- 2 247 172
- US-A- 4 490 145
- US-A- 4 543 097
- US-E- R E29 453

## Description

The present invention relates to ostomy bags that can be confortably worn by the patients.

An ostomy bag is a pouch that is employed to collect waste material that exits a person's body through a stoma, i.e. through an artificial, permanent, opening, surgically created in the ostomate's skin and connected to the intestine or to the bladder.

Colostomy, ileostomy, and urostomy bags, herein collectively referred to as ostomy bags, typically comprise a pouch of plastic, moisture-impermeable and odor-barrier, material; an opening in said bag to allow waste material to enter into the plastic envelope; and means to secure the bag in place with the opening connected to the stoma. This can be obtained by means of a tape that is heat bonded to the ostomy bag around the opening; said tape has an opening which is positioned in-line with the opening of the bag and the side of said tape which is adjacent to the ostomate's body is coated with an adhesive which allows adhesion of the ostomy appliance to the "peristomal area", i.e., the skin area surrounding the stoma.

Preferably the means to secure the ostomy bag to the stoma and the ostomy bag itself, are constructed as two separable parts which are connected and disconnected by the user as desired. This is achieved for instance by applying to the ostomate's skin by means of a suitable adhesive a tape that bears on its outer surface a semi-rigid plastic snap ring, bonded by conventional means to the tape, that surrounds the opening. The ostomy bags then bear a second complementary snap ring mating with the tape one; said complementary snap ring is bonded to the ostomy bag and surrounds the opening therein. The user can therefore apply and remove the ostomy bag from the stoma without peeling off from the skin the means for securing the bag to the stoma. When assembled the snap ring provides a tight leak-free seal.

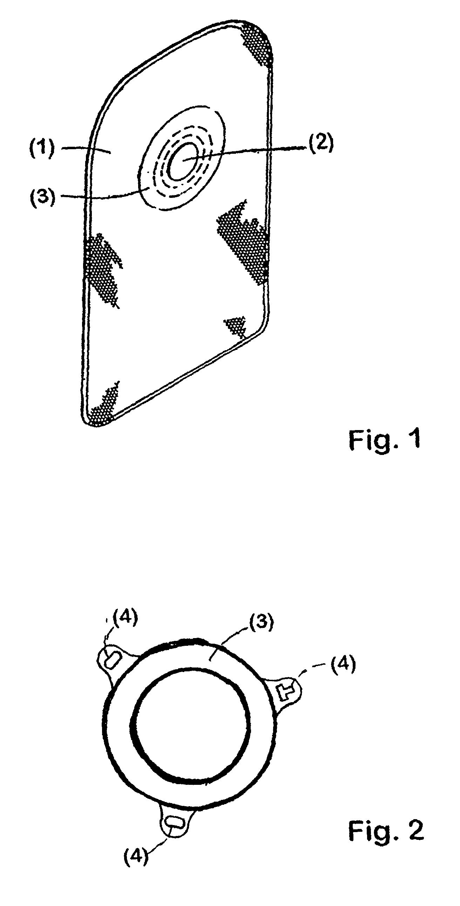

For the sake of clarity, Figures 1 and 2 in the attached drawings illustrate a conventional design for ostomy bags.

In Fig. 1, (1) represents the odor-barrier thermoplastic pouch, (2) is the opening in the pouch that needs to be in-line with the stoma in order to receive the body's waste material, and (3) is the tape that is welded to the pouch and is used to secure the pouch in place by adhering to the ostomate's peristomal skin. In a preferred aspect, as indicated above, the ostomy bag and the means to secure it to the patient's skin are constructed as two separate parts. In such a case, in Fig. 1, (3) represents a ring-shaped flange of semi-rigid plastic as better described in Fig. 2. This pouch strap ring, that preferably has clip retainers (4) spaced thereon, is used to secure the pouch to a tape, applied to the ostomate's skin through its adhesive surface, said last tape bearing a mating ring on its outer surface (not shown in the drawings).

In all events the ostomy bag, which has a surface larger than that of the tape for attachment, lies on the ostomate's skin, conforming the abdominal contours and moving with the skin as the skin moves.

The most common plastic films that are ordinarily employed in the manufacture of ostomy bags, being essentially impermeable to moisture vapor, do not allow the skin to breathe and frequently give rise to irritation of the user's skin, particularly in case of a consistent and prolonged contact therewith.

Different solutions have been proposed to solve this problem, such as the use of a fabric material as the skin contact layer (as in CA-A-1,060,749) or the use of a composite thermoplastic with a skin contact layer of a thermoplastic non-woven material (as in EP-A-433,060).

EP-A-793,951 refers to the use of a perforated polyethylene flocking layer, joined to the ostomy pouch through a peripheral welding, as a comfort backing layer.

A great challenge still exists however to further improve the ostomates' comfort providing ostomy bags that not only meet functional requirements such as good mechanical resistance, easy sealability, and adequate odor barrier properties, but also are as much comfortable for the patient to wear as possible.

It has now been found that by using in the manufacture of an ostomy bag, at least for part of the surface thereof which is in contact with the patient's skin, a thermoplastic material which has been flocked by coating it with an adhesive and then adhering a plurality of individual fiber to said adhesive coating the discomfort which is associated with prolonged contact of moisture impermeable material with the patient's skin is remarkably alleviated.

Flocking (flock coating or flock lining) is a technique that allows the coating of a substrate with a high number of short fibers to give to the finished article a suede- or velvet-like appearance and touch.

In said process, the substrate to be flocked is first coated with a suitable glue or adhesive; then, in an electrostatic field, a great number of individual fibers (flock) are shot vertically into the thick adhesive coating; the flocked surface is then dried and cooled; and the excess flock is sifted out. Electrostatic flocking is based on the law of physics. The rod-shaped flocks take over the charge at one pole (due to either ionization or electrostatic induction) and then travel to the counterpole (the substrate coated with the adhesive) aligning themselves along the lines of force that always enter the substrate vertically.

Flocking machines and production lines for flocking that can be used in the manufacture of the flocked material for the ostomy bag of the present invention are commercially available. An example of a suitable flocking machine is Flocking machine GFS manufactured by Maag Flockmaschinen GmbH.

For the use according to the present invention the substrate or base to be flocked is a thermoplastic mono- or multi-layer film.

Widely differing kinds of fibers can be employed, such as polyamides, polyesters, acrylic polymers, and natural fibers, e.g. cotton and viscose. In a preferred embodiment of the present invention a natural fiber is employed, and even more preferably cotton fibers are used. The length of these fibers is typically less than 5 mm and generally of from about 0.1 to about 3.0 mm. Fibers of from about 0.3 to about 1.5 mm are preferred. The amount of flock fibers distributed per m² of substrate may vary depending on the type of fibers employed, on the line speed and on the more-or-less heavy loading of the substrate which is desired. For the purposes of the present invention, optimum results are obtained with from about 10 to about 100 g/m² and particularly with from about 30 to about 70 g/m² of flock fibers, because a remarkable improvement in hand-feel and appearance is obtained with little increase in weight or stiffness. Fibers of different thickness can also be employed in the manufacture of the ostomy bag according to the present invention. The difference in thickness of the fibers is indicated as the titer which is measured in dtex or grams per 10,000 m length of the individual fiber. For the purpose of the present invention fibers with a titer of from about 2 to about 20 can suitably be employed.

Suitable adhesives will be selected depending on the type of substrate and flock fibers employed. In general said adhesive must be electrically conductive and must remain adhesive on the substrate surface for a time sufficient to allow the vertical fibers to penetrate. It is typically applied to the substrate with a sprayer or by brush, roller, pad or squeegee. Acrylic adhesives, such as polyacrylates or polyacrylates modified with epoxy resins, and urethane adhesives are non limitative examples of suitable adhesives. Alternative adhesives that may suitably be employed include e.g. PVC plastisol adhesives and chlorinated modified polyolefin adhesives.

While the use of a wholly flocked surface in contact with the ostomate's skin provides for optimum comfort of the wearer, it might nevertheless be useful to leave parts of the surface unflocked. As an example leaving small areas of the surface unflocked will allow visual monitoring of the bag contents. Partial flocking can easily be achieved by distributing the adhesive only in the areas to be flocked.

As indicated above, the substrate which is flocked is a mono- or multi-layer thermoplastic film.

In one embodiment of the present invention the substrate is an odor-barrier thermoplastic material, comprising at least one layer of an odor-barrier material, such as PVdC (a copolymer of vinylidene chloride with at least one comonomer copolymerisable therewith), EVOH (ethylene-vinyl alcohol copolymer), PVOH (polyvinyl alcohol), and polyamides or polyamide co- or ter-polymers.

Generally these odor-barrier films comprise, besides the odor-barrier layer, at least one additional outer sealing layer. Generally polyethylenes, linear polyethylenes, ethylene-vinyl acetate copolymers, ethylene-alkyl acrylate copolymers, ethylene-alkyl methacrylate copolymers, polyurethanes or blends thereof are employed for the sealing layer. The polar copolymers and particularly ethylene-vinyl acetate copolymers and polyurethanes, are normally preferred because they are sealable also by Radio Frequency which is a sealing method widely used in this area.

A preferred odor-barrier thermoplastic film that can be flocked and used in the manufacture of ostomy bags according to the present invention comprises three layers, the outer layers comprising an ethylene-vinyl acetate copolymer, preferably with a high content of vinyl acetate units (e.g. 18 or more w. % of VA units), and the core layer being of PVdC. Tie layers may as well be included in this structure, interposed between the core odor-barrier layer and the outer EVA skin layers.

An alternative odor-barrier structure that can suitably be flocked and employed in the manufacture of ostomy bags according to the present invention is a 5-layer structure with a PVdC core layer, polyethylene skin layers and intermediate tie layers.

EVOH, PVOH, and polyamide homo-, co-, or ter-polymer odor-barrier layers may suitably be employed when a chlorine-free structure is desired. Examples of suitable chlorine-free odor-barrier substrates are described for instance in the following patents or patent applications : DE-A-4,100,350, WO-A-93/11938, DD-B-274,386, DD-B-274,387, and EP-A-700,777.

The thickness of the barrier layer as well as that of the other layers in the multi-layer odor-barrier structures to be flocked will be suitably selected, as known in the field, depending on the desired mechanical and odor-barrier properties. In general the thickness of the barrier layer may range from about 4 to about 10 microns, depending on the type of material used to impart odor-barrier properties to the structure. The overall thickness of the substrate will generally range from about 40 to about 200 microns, typically from about 50 to about 130 microns, and preferably from about 60 to about 100 microns.

The flocked odor-barrier substrate can be employed in the manufacture of ostomy bags by methods well known in the art.

In particular, both the skin contact surface and the opposite outer surface of the ostomy bag can be of flocked material. Alternatively the flocked material can be employed for the skin contact surface only, while the outer surface can be made by the same, unflocked, thermoplastic film or by another odor-barrier film, provided the inner surfaces of the two films can be welded together.

The obtained ostomy bag can be provided at the bottom with a drain fitting, such as an integral cap which is opened to drain the bag, when a washable, re-usable, bag is desired.

It is also possible to use the flocked odor-barrier substrate for the manufacture of an outer bag that is used as a cover and holder of an inner, disposable, ostomy pouch.

In such a case the outer flocked bag will provide for the patient's comfort and for at least part of the odor-barrier properties of the overall ostomy appliance.

Said outer bag will be provided with a reclosable cut-out, either horizontally or vertically, generally a slit along one of the bag edges, to allow access of the inner ostomy pouch. The hole of both the outer and the inner bags will be in-line with the stoma, and the outer and inner pouches will be detachably secured one to the other and to the stoma. To take advantage of the odor-barrier properties of the outer flocked bag, the means for closing the outer bag once the inner one is in place as well as the means for securing the two bags together and to the stoma should be designed not to allow passage of air in-between.

As for the reclosable slit, air-tightness can be achieved e.g. by a couple of mating closure strips, positioned on the inner, facing, surfaces of the slitted edges. One strip comprises the male or rib portion which interlocks into the female or grooved portion of the other strip. The ribs and groove portions of the closure strips are easily separable by the fingers of the patient when the filled inner pouch needs to be removed and discarded and can be easily interlocked by snap fitting the male ribs into the corresponding female portion once a new empty inner pouch has been put in place. Alternatively the desired tightness can be provided by an adhesive odor-barrier patch that is used to entirely cover the slit.

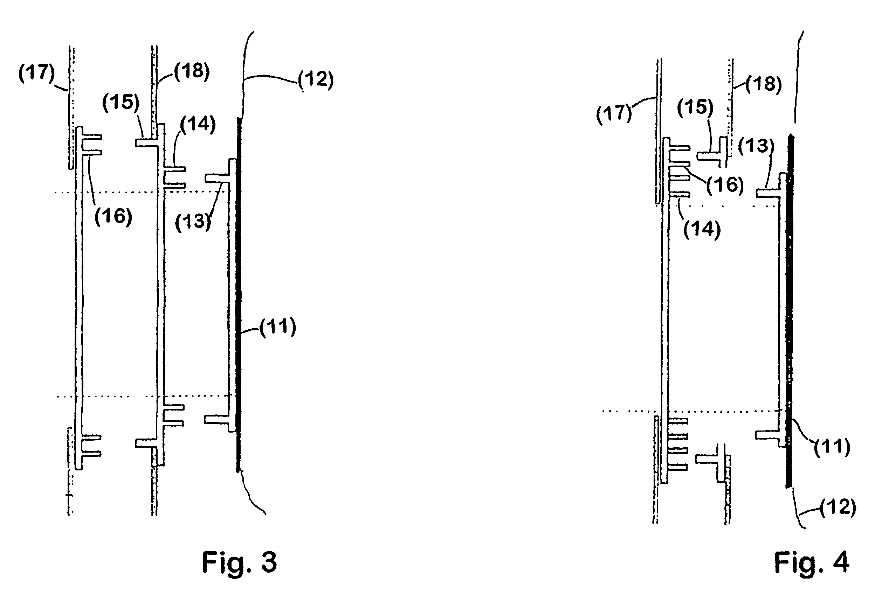

As for the connection of the inner and outer bags to the stoma, Fig. 3 and 4 represent a side view of two different ways of achieving an air-tight, leak-free, connection.

In both cases the overall ostomy appliance is releasably secured to the peristomal area by means of an adhesive tape (11) applied to the patient's body (12), said tape being equipped with a plastic ring (13) that snap fits into a corresponding mating ring (14) positioned on the outer bag in the embodiment of Fig. 3 and on the inner bag in the embodiment of Fig. 4. The system of plastic rings that interlock to tightly secure the inner disposable bag to the outer re-usable one is indicated as (15) and (16). The thermoplastic film of the inner bag is indicated as (17) while (18) is the flocked skin contact surface of the outer bag.

If desired, the ostomy appliance according to the above preferred embodiment of the invention can be more securely attached to the stoma by fastening it to a waist encircling belt, e.g. by means of Velcro patches.

The use of an outer odor-barrier bag allows to decrease the thickness of the odor-barrier layer in the inner, disposable, ostomy pouch. In terms of odor-barrier properties, the results obtained with an ostomy appliance as in said preferred embodiment of the present invention are better than those obtained with a conventional ostomy pouch having an odor-barrier layer thick as the sum of the barrier layers in the separate outer and inner bags.

It would also be possible to use inner chlorine-free ostomy pouches coupled to an outer re-usable PVdC-containing bag. The inner pouches could more safely be disposed of, owing to the absence of a chlorine-containing layer, while the problem of the limited odor-barrier properties of EVOH, PVOH or polyamides would be overcome by the use of an outer bag with better odor-barrier properties. As an example it could be possible to use a toilet flushable PVOH-based inner ostomy pouch, with an outer re-usable bag with a PVdC barrier layer.

Furthermore, since the use of a flocked outer bag, besides increasing the comfort of the wearer, also provides for a sort of protection of the inner bag, the mechanical properties of the inner bag need not be as high as in a conventional ostomy bag and it is therefore possible to decrease the thickness of the film layers in the inner bag. It is thus possible to reduce the amount of plastic waste that needs to be disposed of.

In the ostomy appliance according to the above preferred embodiment, the inner bag might be devoid of an odor-barrier layer. It is however preferred to use an inner bag with an odor-barrier layer at least about 2 micron thick. More preferably, the thickness of said barrier layer in the inner bag would be at least about 3 microns, and typically it is of from about 3 microns to about 5 microns when PVdC is used as the barrier material and from about 3 to about 10 microns when other odor-barrier materials are employed. Depending on the thickness of the odor-barrier layer (if any) in the inner bag, the thickness of the barrier layer in the outer bag may be as low as 2 microns, preferably at least 2.5 microns, typically from about 3 to about 8 microns.

The overall thickness of the inner bag may be reduced down to 35 microns, even if preferably the overall thickness of the inner pouch will be at least 40 microns and typically will be comprised between about 45 and about 75 microns. The overall thickness of the substrate of the outer bag can be as low as 25 microns, but preferably it is comprised between about 35 and about 100 microns.

In another embodiment of the present invention, the substrate to be flocked is not odor-barrier and the obtained material is employed as the outer bag in an ostomy appliance coupled with an inner odor-barrier disposable pouch. In this case, as the outer bag has no odor-barrier properties it is necessary to use an inner ostomy pouch with the desired barrier properties. There would be therefore no purpose and no need to provide in the outer bag for a reclosable slit to insert the inner pouch or for an air-tight connection between the two bags. In this case, as the aim of the outer ostomy bag is only to improve patient's comfort, the outer bag can simply be secured to the inner one by inserting the plastic ring of the inner bag through a hole of suitable size in the outer bag before snap fitting it to the plastic ring of the adhesive tape positioned around the stoma. Furthermore the inner bag can be inserted into the outer one through a slit cut-out, horizontally or vertically, preferably in the surface of the outer bag opposite to the skin contact one. There is no need in such a case to provide for a tight closure of this slit.

In this case the substrate can be any thermoplastic film, either mono- or multi-layer. The thermoplastic material used as the substrate to be flocked is not critical and reasonably cheap resins such as ethylene-vinyl acetate copolymer, polyethylene, linear polyethylene and the like resins can suitably be employed. The thickness of the substrate will generally be comprised between about 20 and about 100 microns.

Also in this case however the thickness of the layers different from the barrier one in the inner ostomy pouch can be reduced due to the lowered demand for mechanical properties. As a consequence thereof also the amount of plastic material that needs to be disposed, will be reduced.

If desired, the outer bag may be fastened to a waist encircling belt as indicated before. Alternatively, in this embodiment of a non-barrier outer bag, said bag may be shaped as a back panel, integral with the belt, with a hole to provide access of the inner pouch hole to the stoma, and a front panel with releasable fastening means for attaching the front panel to the belt, said back and front panels defining a pocket at their lower ends for supporting the inner odor-barrier ostomy pouch, wherein at least the surface of the back panel adjacent to the ostomate's skin is flocked.

## Claims

1. An ostomy bag comprising a bag of thermoplastic material having means to define an opening (2) for receiving waste material from a stoma and means (3) to secure the bag in place with the opening connected to the stoma, **characterized in that** at least a portion of the surface of the bag in contact with the skin of the wearer is flocked by coating it with an adhesive and then adhering a plurality of individual fibers to said adhesive coating.

2. The ostomy bag of claim 1 wherein the whole surface of the bag in contact with the skin of the wearer is flocked.

3. The ostomy bag of claim 1 wherein the fibers used for flocking are selected from polyamide fibers, polyester fibers, fibers of acrylic polymers and natural fibers.

4. The ostomy bag of claim 3 wherein the fibers used for flocking are cotton or viscose fibers.

5. The ostomy bag of any of the preceding claims wherein the length of the fibers is comprised between about 0.1 and about 3.0 mm.

6. The ostomy bag of claim 5 wherein the length of the fibers is of from about 0.3 to about 1.5 mm.

7. The ostomy bag of any of the preceding claims wherein the amount of flock fibers is from about 10 to about 100 g/m².

8. The ostomy bag of claim 7 wherein the amount of flock fibers is from about 30 to about 70 g/m².

9. The ostomy bag of any of the preceding claims wherein the substrate which is flocked is an odor-barrier thermoplastic material.

10. The ostomy bag of claim 9 wherein the odor-barrier thermoplastic material is a multi-layer material comprising at least one layer of an odor-barrier polymer selected from the group consisting of PVdC, EVOH, PVOH, and polyamide homo-, co-, or ter-polymers.

11. The ostomy bag of claim 10 wherein the odor-barrier thermoplastic material comprises two outer layers comprising an ethylene-vinyl acetate copolymer and a core odor-barrier layer of PVdC, and optionally two tie layers interposed between the core odor-barrier layer and the outer layers.

12. The ostomy bag of claim 11 wherein the flock is of cotton or viscose fibers and a polyacrylate adhesive is employed to adhere the fibers to the substrate.

13. An ostomy appliance comprising a re-usable outer ostomy bag and a disposable inner ostomy bag (17) means for securing the two bags together (15), (16) and to the stoma (11) means for inserting and removing the inner bag from the outer one, and means for closing, in an air-tight manner, the outer bag once the inner one is in place (13), (14) **characterized in that** the outer bag is as defined in any of preceding claims 9 to 12.

14. The ostomy appliance of claim 13 wherein a system of semi-rigid plastic rings that interlock each other is employed to secure the two bags together and to the stoma.

15. The ostomy appliance of claim 14 wherein the inner bag is secured to the outer bag and the outer bag is secured to the stoma.

16. The ostomy appliance of claims 14 wherein the inner bag has a twin system of snap fitting rings securing it to the outer bag and to the stoma.

17. An ostomy appliance comprising a re-usable outer ostomy bag and a disposable inner ostomy bag (17), means for securing the two bags together (15),(16) and to the stoma (11), and means for inserting and removing the inner bag from the outer one, **characterized in that** the outer bag is as defined in any of proceding claims 1 to 8 wherein the thermoplastic substrate which is flocked is a non barrier material.

18. The ostomy appliance of claim 17 wherein the thermoplastic substrate which is flocked comprises polyethylene, linear polyethylene, or ethylene-vinyl acetate copolymer.

## Patentansprüche

1. Stomabeutel, der einen Beutel aus thermoplastischem Material (1) mit Mitteln zur Bildung einer Öffnung (2) zur Aufnahme von Ausscheidungsmaterial aus einem Stoma und Mitteln (3) zum Befestigen des Beutels an der gewünschten Stelle umfasst, wobei die Öffnung mit dem Stoma verbunden ist, **dadurch gekennzeichnet, dass** mindestens ein Teil der Oberfläche des Beutels in Kontakt mit der Haut des Trägers durch Beschichten mit Klebstoff und nachfolgendes Ankleben einer Vielzahl individueller Fasern auf die Klebstoffbeschichtung beflockt ist.

2. Stomabeutel nach Anspruch 1, bei dem die gesamte Oberfläche des Beutels in Kontakt mit der Haut des Trägers beflockt ist.

3. Stomabeutel nach Anspruch 1, bei dem die zum Beflocken verwendeten Fasern ausgewählt sind aus Polyamidfasern, Polyesterfasern, Fasern aus Acrylpolymeren und Naturfasern.

4. Stomabeutel nach Anspruch 3, bei dem die zum Beflocken verwendeten Fasern Baumwoll- oder Viskosefasern sind.

5. Stomabeutel nach einem der vorhergehenden Ansprüche, bei dem die Länge der Fasern zwischen etwa 0,1 und etwa 3,0 mm liegt.

6. Stomabeutel nach Anspruch 5, bei dem die Länge der Fasern etwa 0,3 bis etwa 1,5 mm beträgt.

7. Stomabeutel nach einem der vorhergehenden Ansprüche, bei dem die Menge der Beflockungsfasern etwa 10 bis etwa 100 g/m² beträgt.

8. Stomabeutel nach Anspruch 7, bei dem die Menge der Beflokkungsfasern etwa 30 bis etwa 70 g/m² beträgt.

9. Stomabeutel nach einem der vorhergehenden Ansprüche, bei dem das Substrat, welches beflockt wird, thermoplastisches Geruchsbarrierematerial ist.

10. Stomabeutel nach Anspruch 9, bei dem das thermoplastische Geruchsbarrierematerial Mehrschichtmaterial ist, das mindestens eine Schicht aus Geruchsbarrierepolymer ausgewählt aus der Gruppe bestehend aus PVdC, EVOH, PVOH und Polyamidhomo-, -co- oder -terpolymeren umfasst.

11. Stomabeutel nach Anspruch 10, bei dem das thermoplastische Geruchsbarrierematerial zwei Außenschichten, die Ethylen/Vinylacetat-Copolymer umfassen, und eine Geruchsbarriere-Kernschicht aus PVdC und gegebenenfalls zwei Verbindungsschichten umfasst, die zwischen der Geruchsbarriere-Kernschicht und den Außenschichten angeordnet sind.

12. Stomabeutel nach Anspruch 11, bei dem die Beflockung aus Baumwolle- oder Viskosefasern ist und Polyacrylatklebstoff verwendet wird, um die Fasern an das Substrat zu kleben.

13. Stomavorrichtung, die einen wiederverwendbaren äußeren Stomabeutel und einen inneren Einwegstomabeutel (17), Mittel zum Befestigen der beiden Beutel aneinander (15), (16) und an dem Stoma (11), Mittel zum Einsetzen und Entnehmen des inneren Beutels in den bzw. aus dem äußeren Beutel und Mittel zum luftdichten Verschließen des äußeren Beutels umfasst, wenn sich der innere Beutel an der gewünschten Stelle (13), (14) befindet, **dadurch gekennzeichnet, dass** der äußere Beutel wie in einem der vorhergehenden Ansprüche 9 bis 12 definiert ist.

14. Stomavorrichtung nach Anspruch 13, bei der ein System halbstarrer Kunststoffringe, die ineinandergreifen, zum Befestigen der beiden Beutel aneinander und an dem Stoma verwendet wird.

15. Stomavorrichtung nach Anspruch 14, bei der der innere Beutel an dem äußeren Beutel befestigt ist und der äußere Beutel an dem Stoma befestigt ist.

16. Stomavorrichtung nach Anspruch 14, bei der der innere Beutel ein Doppelsystem aus Schnappverbindungsringen aufweist, die ihn an dem äußeren Beutel und an dem Stoma befestigen.

17. Stomavorrichtung, die einen wiederverwendbaren äußeren Stomabeutel und einen inneren Einwegstomabeutel (17), Mittel zum Befestigen der beiden Beutel aneinander (15), (16) und an dem Stoma (11), Mittel zum Einsetzen und Entnehmen des inneren Beutels in den bzw. aus dem äußeren Beutel umfasst, **dadurch gekennzeichnet, dass** der äußere Beutel wie in einem der vorhergehenden Ansprüche 1 bis 8 definiert ist, wobei das thermoplastische Substrat, das beflockt ist, Nicht-Barrierematerial ist.

18. Stomavorrichtung nach Anspruch 17, bei der das thermoplastische Substrat, das beflockt ist, Polyethylen, lineares Polyethylen oder Ethylen/Vinylacetat-Copolymer umfasst.

## Revendications

1. Sachet d'ostomie comprenant un sachet en matériau thermoplastique (1) présentant un moyen pour définir une ouverture (2) pour recevoir des matériaux de déchet d'un stoma, et un moyen (3) pour fixer le sachet en place avec l'ouverture reliée au stoma, **caractérisé en ce qu'**au moins une portion de la surface du sachet en contact avec la peau de l'utilisateur est floquée en la revêtant d'un adhésif et en faisant adhérer ensuite plusieurs fibres individuelles audit revêtement adhésif.

2. Sachet d'ostomie selon la revendication 1, où toute la surface du sachet en contact avec la peau de l'utilisateur est floquée.

3. Sachet d'ostomie selon la revendication 1, où les fibres utilisées pour le flocage sont sélectionnées parmi des fibres de polyamide, des fibres de polyester, des fibres de polymères acryliques et des fibres naturelles.

4. Sachet d'ostomie selon la revendication 3, où les fibres utilisées pour le flocage sont des fibres de coton ou de viscose.

5. Sachet d'ostomie selon l'une des revendications précédentes, où la longueur des fibres est comprise entre environ 0,1 et environ 3,0 mm.

6. Sachet d'ostomie selon la revendication 5, où la longueur des fibres s'étend d'environ 0,3 à environ 1,5 mm.

7. Sachet d'ostomie selon l'une des revendications précédentes, où la quantité de fibres floquées est d'environ 10 à environ 100 g/m².

8. Sachet d'ostomie selon la revendication 7, où la quantité de fibres floquées est d'environ 30 à environ 70 g/m².

9. Sachet d'ostomie selon l'une des revendications précédentes, où le substrat qui est floqué est un matériau thermoplastique formant barrière aux odeurs.

10. Sachet d'ostomie selon la revendication 9, où le matériau thermoplastique formant barrière aux odeurs est un matériau multicouche comprenant au moins une couche en un polymère formant barrière aux odeurs sélectionné dans le groupe constitué de PVdC, de EVOH, de PVOH, et de homo-,co- ou ter-polymères de polyamide.

11. Sachet d'ostomie selon la revendication 10, où le matériau thermoplastique formant barrière aux odeurs comprend deux couches externes comprenant un copolymère éthylène/acétate de vinyle et une couche centrale formant barrière aux odeurs en PVdC, et en option deux couches de liaison interposées entre la couche centrale formant barrière aux odeurs et les couches externes.

12. Sachet d'ostomie selon la revendication 11, où les flocons sont en coton ou en fibres de viscose, et un adhésif en polyacrylate est utilisé pour faire adhérer les fibres au substrat.

13. Appareil d'ostomie comprenant un sachet d'ostomie extérieur réutilisable et un sachet d'ostomie intérieur jetable (17), un moyen pour fixer les deux sachets ensemble (15, 16) et au stoma (11), un moyen pour insérer et retirer le sachet interne du sachet externe, et un moyen pour fermer, d'une manière étanche à l'air, le sachet externe lorsque le sachet interne est en place (13, 14), **caractérisé en ce que** le sachet externe est tel que défini dans l'une des revendications précédentes 9 à 12.

14. Appareil d'ostomie selon la revendication 13, où un système de bagues plastiques semi-annulaires qui s'interverrouillent, est utilisé pour fixer les deux sachets ensemble et au stoma.

15. Appareil d'ostomie selon la revendication 14, où le sachet interne est fixé au sachet externe, et le sachet externe est fixé au stoma.

16. Appareil d'ostomie selon la revendication 14, où le sachet interne comporte un système double de bagues à enclenchement, le fixant au sachet externe et au stoma.

17. Appareil d'ostomie comprenant un sachet d'ostomie extérieur réutilisable et un sachet d'ostomie intérieur jetable (17), un moyen pour fixer les deux sachets l'un à l'autre (15, 16) et au stoma (11) et un moyen pour insérer et retirer le sachet interne du sachet externe, **caractérisé en ce que** le sachet externe est tel que défini dans l'une des revendications précédentes 1 à 8, où le substrat thermoplastique qui est floqué, est un matériau ne formant pas barrière.

18. Appareil d'ostomie selon la revendication 17, où le substrat thermoplastique qui est floqué comprend du polyéthylène, du polyéthylène linéaire ou un copolymère éthylène-acétate de vinyle.
